# EUROPÄISCHE PATENTSCHRIFT

(11) **EP 3 206 657 B1**
(45) Veröffentlichungstag und Bekanntmachung des Hinweises auf die Patenterteilung: **07.11.2018**
(21) Anmeldenummer: 15757518.4
(22) Anmeldetag: 08.09.2015
(51) Int. Cl.: A61K 8/06, A61K 8/26, A61K 8/37, A61Q 15/00

(54) **ANTI-TRANSPIRANTIEN MIT ALUMINIUMSESQUICHLORHYDRAT UND CITRONENSÄURETRIETHYLESTER**
ANTI-TRANSPIRANTS WITH ALUMINUMSESQUICHLOROHYDRAT UND CITRIC ACID TRIETHYLESTER
ANTI-PERSPIRANT AVEC ALUMINUMSESQUICHLOROHYDRAT ET ACIDE CITRIQUE TRIÉTHYLIQUE

(30) Priorität: 15.10.2014 DE 102014220918
(43) Veröffentlichungstag der Anmeldung: 23.08.2017
(73) Patentinhaber: Henkel AG & Co. KGaA, 40589 Düsseldorf (DE)
(72) Erfinder: DÖRING, Thomas, 41540 Dormagen (DE)
(86) Internationale Anmeldenummer: PCT/EP2015/070436
(87) Internationale Veröffentlichungsnummer: WO 2016/058752

(56) Entgegenhaltungen:
- WO-A1-01/07001
- WO-A1-98/17241
- DE-A1- 19 917 743
- DE-A1-102012 222 692
- US-A1- 2014 173 833

## Beschreibung

Die vorliegende Anmeldung betrifft ein antitranspirantes kosmetisches Mittel mit niedrigem pH-Wert in Form einer wässrigen Emulsion, welches Aluminiumsesquichlorhydrat und Citronensäuretriethylester enthält.

Die vorliegende Erfindung betrifft weiterhin ein nicht-therapeutisches kosmetisches Verfahren zur Behandlung des menschlichen Körpers, insbesondere zur Verhinderung und/oder Reduzierung der Transpiration des Körpers, bei welchem das erfindungsgemäße schweißhemmende Mittel auf die Haut, insbesondere auf die Haut der Achselhöhlen, aufgetragen wird.

Weiterhin betrifft die vorliegende Erfindung die Verwendung einer Kombination von Aluminiumsequichlorhydrat und Citronensäureethylester, wobei diese in einer wässrigen Emulsion mit einem pH-Wert von 3,5 bis 4,0 vorliegen, zur Reduzierung und/oder Verhinderung von Schweiß, insbesondere Achselschweiß.

Das Waschen, Reinigen und Pflegen des eigenen Körpers stellt ein menschliches Grundbedürfnis dar, und die moderne Industrie versucht fortlaufend, diesen Bedürfnissen des Menschen in vielfältiger Weise gerecht zu werden. Besonders wichtig für die tägliche Hygiene ist die anhaltende Beseitigung oder zumindest Reduzierung des Körpergeruchs. Im Stand der Technik sind zahlreiche spezielle deodorierende oder schweißhemmende Körperpflegemittel bekannt, die für die Anwendung in Körperregionen mit einer hohen Dichte von Schweißdrüsen, insbesondere im Achselbereich, entwickelt wurden. Diese sind in den unterschiedlichsten Darreichungsformen konfektioniert, beispielsweise als Puder, in Stiftform, als Aerosolspray, Pumpspray, flüssige und gelförmige Roll on-Applikation, Creme, Gel und als getränktes flexibles Substrat (Deotücher).

Kosmetische Antitranspirantien enthalten meistens mindestens ein Öl oder eine Fettsubstanz, eine Riechstoffkomponente bzw. ein Parfüm und mindestens ein schweißhemmendes Salz.

Die in Antitranspirantien eingesetzten schweißhemmenden Salze verringern zum einen die Schweißsekretion des Körpers durch eine temporäre Verengung oder Verstopfung der Ausführungsgänge der Schweißdrüsen, so dass die Schweißmenge um etwa 20 bis 60 Prozent reduziert werden kann. Zum anderen weisen sie aufgrund ihrer antimikrobiellen Wirkung einen zusätzlichen desodorierenden Effekt auf.

Als schweißhemmendes Salz werden für gewöhnlich aktivierte basische Aluminium- und Aluminium-

Zirkoniumhalogenide, wie in den Druckschriften EP 0 308 937 A2, EP 0 183 171 A2, US 4 359 456 A und EP 0 191 628 beschrieben, eingesetzt. Weiterhin können auch Aluminium- und Aluminium-Zirkoniumhalogenide eingesetzt werden, welche mit organischen Säuren als Komplexliganden stabilisiert sind, wie sie beispielsweise in den Druckschriften US 3 542 919 A, US 3 553 316 A, US 3 991 176 A und WO 2005/092795 A1 offenbart sind.

WO 2014/083175 A1 und US 2005/0163737 A1 offenbaren neben weiteren Aluminiumsalzen die Verwendung verschiedener Aluminiumchlorhydrate und von Derivaten davon als schweißhemmende Bestandteile von Antitranspirantien, u.a. auch Aluminumsesquichlorhydrat. Reguläres Aluminiumchlorhydrat hat üblicherweise ein molares Al/Cl-Verhältnis von etwa 1,9:1 bis 2,1:1. Aluminumsesquichlorhydrat hat ein geringeres molares Al/Cl-Verhältnis, häufig von etwa 1,5:1 bis 1,8:1. Aluminiumsesquichlorhydrat reagiert in wässriger Lösung saurer als Aluminiumchlorhydrat.

Aluminiumchlorhydrat und andere Aluminiumsalze, die als antitranspirant wirkende Komponenten eingesetzt werden, erfordern üblicherweise hohe Dosierungen von etwa 20 Gew.-% des antitranspiranten Mittels. Die Folge sind unerwünschte Rückstände auf der Haut und Flecken auf Textilien sowie klebrige Sensorik und Hautunverträglichkeiten.

Der pH-Wert der Hautoberfläche liegt üblicherweise bei etwa pH 5,5. Um die reizende Wirkung der Aluminiumsalze wie Aluminiumchlorhydrat auf die Hautoberfläche nicht noch zu steigern, ist der pH handelsüblicher antitranspiranter Zusammensetzungen üblicherweise auf einen Bereich größer als pH 4,0 bis etwa 4,5 eingestellt, also einen pH-Wert, der nicht allzu weit vom pH-Wert der Hautoberfläche entfernt ist.

Aufgabe der vorliegenden Erfindung war es, ein antitransparentes Mittel bereitzustellen, welches eine geringe Dosierung eines Aluminiumsalzes als antitranspirantem Wirkstoff erlaubt, wobei eine sehr gute antitranspirante Wirkung erzielt wird und Hautunverträglichkeiten sowie Rückstände auf Haut und Flecken auf Textilien möglichst vermieden werden können.

Es wurde nun gemäß dieser Erfindung überraschend festgestellt, dass dies durch ein antitranspirantes kosmetisches Mittel erreicht werden kann, welches eine Kombination von Aluminiumsesquichlorhydrat und Citronensäuretriethylester enthält, wobei das Mittel als wässrige Emulsion formuliert ist und einen pH-Wert von 3,5 bis 4,0 aufweist.

Ähnliche Mittel werden in DE102012222692 A1 und WO01/07001 A1 offenbart. Durch die vorliegende Anmeldung wird bereitgestellt:
1. Ein antitranspirantes kosmetisches Mittel in Form einer wässrigen Emulsion, welches enthält:
   - Aluminiumsesquichlorhydrat und
   - Citronensäuretriethylester,
   wobei das kosmetische Mittel einen pH-Wert von 3,5 bis 4,0 aufweist und wobei ferner
   das Aluminiumsesquichlorhydrat in einer Menge von 5,0 bis 15,0 Gew.-% enthalten ist und der Citronensäuretriethylester in einer Menge von 0,1 bis 7 Gew.-% enthalten ist, wobei sich die Gew.-%-Angaben jeweils auf das Gesamtgewicht des erfindungsgemäßen Mittels beziehen, ohne ggf. vorhandenes Treibmittel zu berücksichtigen.
   Zwingend erforderlich im Sinne der vorliegenden Erfindung ist ferner, dass das Aluminiumsesquichlorhydrat ein molares Al/ Cl-Verhältnis von 1,5:1 bis 1,8:1 aufweist.
2. Kosmetisches Mittel nach Punkt 1 oder 2, wobei das Aluminiumsesquichlorhydrat in einer Menge von 8,0 bis 12,0 Gew.-% enthalten ist und der Citronensäuretriethylester in einer Menge von 0,5 bis 2 Gew.-% enthalten ist, wobei sich die Gew.-%-Angaben jeweils auf das Gesamtgewicht des erfindungsgemäßen Mittels beziehen, ohne ggf. vorhandenes Treibmittel zu berücksichtigen.
3. Kosmetisches Mittel nach einem der vorhergehenden Punkte, wobei das kosmetische Mittel kein Ethylhexylglycerin enthält.
4. Kosmetisches Mittel nach einem der vorhergehenden Punkte, wobei das kosmetische Mittel neben dem Aluminiumsesquichlorhydrat und dem Citronensäuretriethylester keine weiteren als Antitranspirans wirkenden Komponenten enthält.
5. Kosmetisches Mittel nach einem der vorhergehenden Punkte, wobei das kosmetische Mittel kein Dipropylenglykol enthält.
4. Kosmetisches Mittel nach einem der vorhergehenden Punkte, wobei das kosmetische Mittel kein Propylenglykol und kein Dipropylenglykol enthält.
5. Kosmetisches Mittel nach einem der vorhergehenden Punkte, wobei das kosmetische Mittel weiterhin Phenoxyethanol enthält.
6. Kosmetisches Mittel nach einem der vorhergehenden Punkte, wobei das kosmetische Mittel als Öl-in-Wasser-Emulsion vorliegt.
7. Kosmetisches Mittel nach einem der vorhergehenden Punkte, wobei der pH-Wert des kosmetischen Mittels 3,5 bis 3,95, bevorzugt 3,6 bis 3,9, ebenfalls bevorzugt 3,7 bis 3,8, beträgt.
8. Verfahren zur nicht-therapeutischen, kosmetischen schweißhemmenden Behandlung des Körpers, bei dem ein antitranspirantes kosmetisches Mittel nach einem der Punkte 1 bis 11 auf die Haut, insbesondere die Haut der Achselhöhlen, aufgebracht wird.
9. Nichttherapeutische, kosmetische Verwendung einer wässrigen Emulsion, welche eine Kombination von Aluminiumsequichlorhydrat und Citronensäureethylester enthält, wobei die wässrige Emulsion einen pH-Wert von 3,5 bis 4,0 aufweist, zur Reduzierung und/oder Verhinderung von Schweiß, insbesondere Achselschweiß.

Bezüglich bevorzugter Ausführungsformen des erfindungsgemäßen Verfahrens und der erfindungsgemäßen Verwendung gilt mutatis mutandis das zu den erfindungsgemäßen Mitteln Gesagte.

Überraschenderweise wurde gefunden, dass bei einer Kombination des spezifischen antitranspiranten Wirkstoffs Aluminiumsesquichlorhydrat, mit dem zwingend erforderlichen molaren Al/Cl-Verhältnis von 1,5:1 bis 1,8:1, mit Citronensäuretriethylester (INCI: Triethyl Citrate) eine besonders hohe schweißhemmende Wirkung erzielt werden kann. Dies erlaubt die Einbeziehung von Aluminiumsesquichlorhydrat in einer vergleichsweise geringen Menge von bspw. 8 bis 12 Gew.-%, bezogen auf das Gesamtgewicht des antitranspiranten Mittels, ohne ggf. vorhandenes Treibmittel zu berücksichtigen. Offenbar wirken diese beiden Komponenten in synergistischer Weise zusammen. Ohne auf eine Theorie festgelegt werden zu wollen, wird vermutet, dass sich bei dieser Kombination von Komponenten besonders wirksame Komplexe des Aluminiums ausbilden.

Bei Anwendung des erfindungsgemäßen antitranspiranten Mittels wurde eine sehr gute Hautverträglichkeit, wenige Rückstände und Flecken auf der Haut und Textilien und ein verbessertes, weniger klebriges Hautgefühl beobachtet.

Weiterhin wurde bei Anwendung des erfindungsgemäßen kosmetischen Mittels auf der Hautoberfläche eine Absenkung des pH-Werts der Haut beobachtet. In der Regel wird bei Aufbringen des erfindungsgemäßen kosmetischen Mittels auf die Haut eine Verringerung des pH-Werts der Haut um etwa 0,2 bis 0,7 innerhalb von 60 Minuten beobachtet.

Die Komponenten Aluminiumsesquichlorhydrat und Citronensäuretriethylester wirken synergistisch so gut zusammen, dass gemäß Ausführungsformen der Erfindung weitere als Antitranspirans wirkende Komponenten nicht enthalten sein brauchen. Derartige Zusammensetzungen, die keine weiteren, als Antitranspirans wirkenden Komponenten enthalten, sind erfindungsgemäß ausdrücklich umfasst.

In Ausführungsformen der Erfindung enthält das antitranspirante kosmetische Mittel kein Ethylhexylglycerin. In weiteren Ausführungsformen der Erfindung enthält das antitranspirante kosmetische Mittel kein Dipropylenglykol. Dipropylenglykol kann die schweißhemmende Wirkung der kosmetischen Zusammensetzung der Erfindung beeinträchtigen und ist daher in einer bevorzugten Ausführungsform nicht enthalten. In weiteren Ausführungsformen der Erfindung sind kein Dipropylenglykol und kein Propylenglykol in dem kosmetischen Mittel enthalten.

Selbstverständlich sind erfindungsgemäß aber auch Ausführungsformen umfasst, bei denen weitere als Antitranspirans wirkende Komponenten enthalten sind, um die antitranspirante Wirkung des erfindungsgemäßen kosmetischen Mittels ggf. noch zu verstärken. Weitere mögliche Bestandteile des erfindungsgemäßen kosmetischen Mittels werden weiter unten erläutert.

Ein notwendiger Bestandteil des erfindungsgemäßen antitranspiranten Mittels ist Aluminiumsesquichlorhydrat. Aluminiumsesquichlorhydrat kann allgemein durch die Formel Al₂Clₐ(OH)₆₋ₐ beschrieben werden, z.B. Al₂(OH)₄Cl_{1,5} · xH₂0, wobei diese Formeln die ungefähre Zusammensetzung angeben. Erfindungsgemäß liegt a im Bereich von 1,33 bis 1,1, sodass das Aluminiumsesquichlorhydrat dann ein molares Al/Cl-Verhältnis von 1,5:1 bis etwa 1,8:1 aufweist.

Das Aluminiumsesquichlorhydrat ist in dem erfindungsgemäßen antitranspiranten Mittel bevorzugt in einem Anteil von 5,0 bis 15,0 Gew.-%, bevorzugter 6,0 bis 14,0 Gew.-%, weiter bevorzugt 7,0 bis 13,0 Gew.-% und noch bevorzugter 8,0 bis 12, 0 Gew.-%, enthalten, wobei sich die Gew.-%-Angaben jeweils auf das Gesamtgewicht des erfindungsgemäßen Mittels beziehen, ohne ggf. vorhandenes Treibmittel zu berücksichtigen.

Ein weiterer notwendiger Bestandteil des erfindungsgemäßen antitranspiranten Mittels ist Citronensäuretriethylester (Triethylcitrat). Triethylcitrat ist ein bekannter Deodorantwirkstoff. Bevorzugte erfindungsgemäße Mittel enthalten 0,1 - 7,0 Gew.-% Citronensäuretriethylester, bevorzugter 0,5 - 5,0 Gew.-%, weiter bevorzugt 0,5 bis 3,0 Gew.-%, am bevorzugtesten 1,0 bis 2,0 Gew.%, jeweils bezogen auf das Gewicht der Zusammensetzung, ohne ggf. vorhandenes Treibmittel zu berücksichtigen.

Das erfindungsgemäße kosmetische Mittel enthält die Komponenten Aluminiumsesquichlorhydrat und Triethylcitrat in Kombination bevorzugt in folgenden Anteilen:
Aluminiumsesquichlorhydrat in einer Menge von 5,0 bis 15,0 Gew.-%, bevorzugter 8,0 bis 12,0 Gew.-%, und Citronensäuretriethylester in einer Menge von 0,1 bis 7,0 Gew.-%, bevorzugter 0,5 bis 2,0 Gew.-%, wobei sich die Gew.-%-Angaben jeweils auf das Gesamtgewicht des erfindungsgemäßen Mittels beziehen, ohne ggf. vorhandenes Treibmittel zu berücksichtigen.

Handelsübliche antitranspirante Mittel, die Aluminiumsalze als wirksame Komponenten enthalten, weisen wie erwähnt einen pH größer 4,0 auf, wobei in der Regel ein höherer pH angestrebt wird, um sich dem pH der Hautoberfläche anzunähern und dadurch Hautreizungen zu minimieren.
Es war daher überraschend, dass bei einer Kombination der Komponenten Citronensäuretriethylester und Aluminiumsesquichlorhydrat in einer wässrigen Emulsion mit einem pH von 3,5 bis 4,0 einerseits eine hervorragende antitranspirante Wirkung erzielt und gleichzeitig Hautunverträglichkeiten minimiert werden konnten.
Der pH-Wert der wässrigen Emulsion bzw. des antitranspiranten Mittels in der vorliegenden Erfindung ist bevorzugt 3,5 bis 3,95, bevorzugter 3,6 bis 3,9, ebenfalls bevorzugt 3,7 bis 3,8. Der pH Wert wird dabei erfindungsgemäß mit dem pH Meter pH730 von inoLab bei Normalbedingungen gemessen.

Das erfindungsgemäße kosmetische Mittel liegt als wässrige Emulsion vor, bevorzugt als Öl-in-Wasser-Emulsion. In diesem Fall wird das erfindungsgemäße Mittel bevorzugt als treibmittelfreies Pumpspray oder Quetschspray versprüht oder als Roll-on appliziert.

In einer weiteren bevorzugten Darreichungsform liegt das schweißhemmende kosmetische Mittel als Wasser-in-ÖI-Emulsion vor. Hierbei kann es sich insbesondere um eine versprühbare Wasser-in-ÖI-Emulsion handeln, welche mittels eines Treibmittels versprüht werden kann.

Bei der Ölkomponente handelt es sich um ein kosmetisches Öl. Wenn in dieser Erfindung von einem kosmetischen Öl die Rede ist, handelt es sich hierbei immer um ein kosmetisches Öl, das kein Riechstoff und kein ätherisches Öl ist, unter Normalbedingungen flüssig und mit Wasser nicht mischbar ist. Als kosmetische Öle kommen flüchtige kosmetische Öle und nichtflüchtige kosmetische Öle in Frage.

Der Begriff "flüchtiges kosmetisches Öl" bezeichnet erfindungsgemäß kosmetische Öle, welche bei 20°C und einem Umgebungsdruck von 1.013 hPa einen Dampfdruck von 2,66 Pa bis 40.000 Pa (0,02 bis 300 mm Hg), vorzugsweise von 10 bis 12.000 Pa (0,1 bis 90 mm Hg), weiter bevorzugt von 13 bis 3.000 Pa (0,1 bis 23 mm Hg), insbesondere von 15 bis 500 Pa (0,1 bis 4 mm Hg), aufweisen.

Darüber hinaus werden unter dem Begriff "nichtflüchtige kosmetische Öle" im Sinne der vorliegenden Erfindung kosmetische Öle verstanden, welche bei 20°C und einem Umgebungsdruck von 1.013 hPa einen Dampfdruck von weniger als 2,66 Pa (0,02 mm Hg) aufweisen.

Gemäß einer bevorzugten Ausführungsform der vorliegenden Erfindung ist das bei 20°C und 1.013 hPa flüssige kosmetische Öl ausgewählt aus der Gruppe von
(i) flüchtigen cyclischen Siliconölen, insbesondere Cyclotrisiloxan, Cyclotetrasiloxan, Cyclopentasiloxan und Cyclohexasiloxan, und linearen Siliconölen mit 2 bis 10 Siloxaneinheiten, insbesondere Hexamethyldisiloxan, Octamethyltrisiloxan, Decamethyltetrasiloxan;
(ii) flüchtigen Nichtsiliconölen, insbesondere flüssigen Paraffinölen und Isoparaffinölen, wie Isodecan, Isoundecan, Isododecan, Isotridecan, Isotetradecan, Isopentadecan, Isohexadecan und Isoeicosan;
(iii) nichtflüchtigen Siliconölen, insbesondere höhermolekularen linearen Polyalkylsiloxane;
(iv) nichtflüchtigen Nichtsiliconölen, insbesondere den Estern von linearen oder verzweigten gesättigten oder ungesättigten C₂-C₃₀-Fettalkoholen mit linearen oder verzweigten gesättigten oder ungesättigten C₂-C₃₀-Fettsäuren, welche hydroxyliert sein können, den C8-C24-Fettalkoholestern einwertiger oder mehrwertiger C₂-C₇-Hydroxycarbonsäuren, den verzweigten gesättigten oder ungesättigten C₆-C₃₀-Fettalkoholen, den Mono-, Di- und Triglyceriden von linearen oder verzweigten, gesättigten oder ungesättigten, gegebenenfalls hydroxylierten C₈-C₃₀-Fettsäuren, den Dicarbonsäureestern von linearen oder verzweigten C₂-C₁₀-Alkanolen, den Anlagerungsprodukten von Ethylenoxid und/oder Propylenoxid an ein- oder mehrwertige C₃-C₂₂-Alkanole, welche gegebenenfalls verestert sein können, den symmetrischen, unsymmetrischen oder cyclischen Estern der Kohlensäure mit Fettalkoholen, den Estern von Dimeren ungesättigter C₁₂-C₂₂-Fettsäuren mit einwertigen, linearen, verzweigten und cyclischen C₂-C₁₈-Alkanolen oder C₂-C₆-Alkanolen, den Benzoesäureestern von linearen oder verzweigten C₈-C₂₂-Alkanolen, wie Benzoesäure-C₁₂-C₁₅-Alkylester und Benzoesäureisostearylester und Benzoesäureoctyldodecylester, den synthetischen Kohlenwasserstoffen, wie Polyisobuten und Polydecene, den alicyclischen Kohlenwasserstoffen; sowie
(v) deren Mischungen.

Der Einsatz von flüchtigen Siliconölen und flüchtigen Nichtsiliconölen in den erfindungsgemäßen schweißhemmenden kosmetischen Mitteln resultiert in einem trockeneren Hautgefühl und in einer schnelleren Freisetzung des schweißhemmenden Aluminiumsalzes.

Die im Rahmen der Erfindung einsetzbaren cyclischen flüchtigen Siliconöle weisen bei 20 °C und einem Umgebungsdruck von 1.013 hPa einen Dampfdruck von 13 bis 15 Pa (0,1 mm Hg) auf. Weiterhin kann erfindungsgemäß als lineares flüchtiges Siliconöl auch ein niedermolekulares Phenyl Trimethicone mit einem Dampfdruck von etwa 2.000 Pa (15 mm Hg) bei 20°C und einem Umgebungsdruck von 1.013 hPa eingesetzt werden. Aufgrund der hohen Persistenz von Cyclodimethiconen in der Umwelt kann es erfindungsgemäß jedoch bevorzugt sein, wenn in den erfindungsgemäßen schweißhemmenden kosmetischen Mitteln 0 bis weniger als 1 Gew.-%, vorzugsweise 0 bis weniger als 0,1 Gew.-%, cyclische flüchtige Siliconöle eingesetzt werden.

Erfindungsgemäß werden bevorzugt flüchtige Nichtsiliconöle in Form von C 10-13-Isoparaffin-Mischungen mit einem Dampfdruck von 10 bis 400 Pa (0,08 bis 3 mm Hg), vorzugsweise von 13 bis 100 Pa (0,1 bis 0,8mm Hg), bei 20 °C und einem Umgebungsdruck von 1.013 hPa eingesetzt. Dabei ist es im Rahmen der vorliegenden Erfindung bevorzugt, wenn das flüchtige C8-C16-Isoparaffin in einer Gesamtmenge von 1 bis 60 Gew.-%, vorzugsweise von 3 bis 45 Gew.-%, bevorzugt von 5 bis 40 Gew.-%, insbesondere von 8 bis 20 Gew.-%, bezogen auf das Gesamtgewicht des schweißhemmenden kosmetischen Mittels, enthalten ist. Selbstverständlich ist es ebenfalls möglich, erfindungsgemäße schweißhemmende kosmetische Mittel mit einem geringen Anteil an flüchtigen Ölen - das heißt, mit 0,5 bis 15 Gew.-%, bezogen auf das Gesamtgewicht des schweißhemmenden kosmetischen Mittels, an flüchtigen Ölen - oder sogar ohne flüchtige Öle zu formulieren.

Zur Maskierung von unlöslichen Bestandteilen, wie Talkum oder auf der Haut angetrockneten schweißhemmenden Aluminiumsalzen, kann es erfindungsgemäß bevorzugt sein, wenn die schweißhemmenden kosmetischen Mittel ein nichtflüchtiges Siliconöl und/oder ein nichtflüchtiges Nichtsiliconöl enthalten.

In diesem Zusammenhang enthalten erfindungsgemäß bevorzugte schweißhemmende kosmetische Mittel mindestens einen Ester der linearen oder verzweigten gesättigten oder ungesättigten Fettalkohole mit 2 bis 30 Kohlenstoffatomen mit linearen oder verzweigten gesättigten oder ungesättigten Fettsäuren mit 2 bis 30 Kohlenstoffatomen, welche hydroxyliert sein können, in einer Gesamtmenge von 1 bis 30 Gew.-%, vorzugsweise von 5 bis 26 Gew.-%, bevorzugt von 9 bis 24 Gew, bezogen auf das Gesamtgewicht des schweißhemmenden kosmetischen Mittels.

Im Rahmen der vorliegenden Erfindung können als nichtflüchtige Siliconöle lineare Polyalkylsiloxane mit einer kinematischen Viskosität bei 25°C von 5 bis 2.000 cSt, insbesondere linearen Polydimethylsiloxane mit einer kinematischen Viskosität bei 25°C von 5 bis 2.000 cSt, vorzugsweise von 10 bis 350 cSt, insbesondere von 50 bis 100 cSt, eingesetzt werden. Die vorstehend genannten nichtflüchtigen Siliconöle sind unter den Handelsnamen Dow Corning® 200 bzw. Xiameter PMX von Dow Corning bzw. Xiameter erhältlich. Weitere bevorzugte nichtflüchtige Siliconöle sind Phenyltrimethicone mit einer kinematischen Viskosität bei 25°C von 10 bis 100 cSt, vorzugsweise von 15 bis 30 cSt sowie Cetyldimethicone.

Erfindungsgemäß weiterhin bevorzugt ist der Einsatz von Mischungen der vorstehend genannten kosmetischen Öle, insbesondere von nichtflüchtigen und flüchtigen kosmetischen Ölen, da auf diese Weise Parameter wie Hautgefühl, Sichtbarkeit des Rückstands und Stabilität des erfindungsgemäßen schweißhemmenden kosmetischen Mittels eingestellt und das Mittel somit besser an die Bedürfnisse der Verbraucher angepasst werden kann.

Im Rahmen der vorliegenden Erfindung ist es bevorzugt, wenn das bei 20°C und 1.013 hPa flüssige kosmetische Öl in einer Gesamtmenge von 1 bis 95 Gew.-%, vorzugsweise von 10 bis 85 Gew.-%, bevorzugt von 20 bis 75 Gew.-%, weiter bevorzugt von 35 bis 70 Gew.-%, bezogen auf das Gesamtgewicht des schweißhemmenden kosmetischen Mittels, enthalten ist.

Es kann jedoch auch bevorzugt sein, wenn das bei 20°C und 1.013 hPa flüssige kosmetische Öl in einer Gesamtmenge von 0,2 bis 70 Gew.-%, vorzugsweise von 2 bis 60 Gew.-%, bevorzugt von 3 bis 50 Gew.-%, weiter bevorzugt von 5 bis 35 Gew.-%, insbesondere von 8 bis 20 Gew.-%, bezogen auf das Gesamtgewicht des schweißhemmenden kosmetischen Mittels, enthalten ist.

Es sei an dieser Stelle darauf hingewiesen, dass sich die Angabe Gew.-%, sofern es nicht anders angegeben ist, jeweils auf das Gesamtgewicht des erfindungsgemäßen Mittels bezieht, ohne ggf. vorhandenes Treibmittel zu berücksichtigen.

Die Begriffe "erfindungsgemäßes (antitranpirantes oder kosmetisches) Mittel" und "erfindungsgemäße Zusammensetzung" werden in der vorliegenden Anmeldung synonym gebraucht.

"Normalbedingungen" sind im Sinne der vorliegenden Anmeldung eine Temperatur von 20 °C und ein Druck von 1013,25 mbar. Schmelzpunktangaben beziehen sich ebenfalls auf einen Druck von 1013,25 mbar.

Die erfindungsgemäßen schweißhemmenden kosmetischen Mittel können darüber hinaus weitere Hilfsstoffe enthalten. Bevorzugt enthalten die erfindungsgemäßen schweißhemmenden kosmetischen Mittel mindestens einen weiten Hilfsstoff, ausgewählt aus der Gruppe von (i) Emulgatoren und/oder Tensiden; (ii) Hydrogelbildnern; (iii) Chelatbildnern; (iv) Deodorant-Wirkstoffen; (v) ein- und/oder mehrwertigen Alkoholen und/oder Polyethylenglycolen; (vi) hautkühlenden Wirkstoffen; (vii) Treibmitteln; (viii) Verdickungsmitteln sowie (ix) deren Mischungen.

Erfindungsgemäß bevorzugt geeignete Emulgatoren und Tenside sind ausgewählt aus anionischen, kationischen, nichtionischen, amphoteren, insbesondere ampholytischen und zwitterionischen Emulgatoren und Tensiden. Tenside sind amphiphile (bifunktionelle) Verbindungen, die aus mindestens einem hydrophoben und mindestens einem hydrophilen Molekülteil bestehen. Der hydrophobe Rest ist bevorzugt eine Kohlenwasserstoffkette mit 8 bis 28 Kohlenstoffatomen, die gesättigt oder ungesättigt, linear oder verzweigt sein kann. Besonders bevorzugt ist diese C8-C28-Alkylkette linear.

Unter anionischen Tensiden werden Tenside mit ausschließlich anionischen Ladungen verstanden; sie enthalten z. B. Carboxylgruppen, Sulfonsäuregruppen oder Sulfatgruppen. Besonders bevorzugte anionische Tenside sind Alkylsulfate, Alkylethersulfate, Acylglutamate und C8-24-Carbonsäuren sowie deren Salze, die sogenannten Seifen. Unter kationischen Tensiden werden Tenside mit ausschließlich kationischen Ladungen verstanden; sie enthalten z. B. quartäre Ammoniumgruppen. Bevorzugt sind kationische Tenside vom Typ der quartären Ammoniumverbindungen, der Esterquats und der Amidoamine. Bevorzugte quaternäre Ammoniumverbindungen sind Ammoniumhalogenide sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Die amphoteren Tenside werden in ampholytische Tenside und zwitterionische Tenside unterteilt. Unter ampholytischen Tensiden werden solche oberflächenaktiven Verbindungen verstanden, die sowohl saure (beispielsweise -COOH oder - SO₃H-Gruppen) als auch basische hydrophile Gruppen (beispielsweise Aminogruppen) besitzen und sich also je nach Bedingung sauer oder basisch verhalten. Unter zwitterionischen Tensiden versteht der Fachmann Tenside, die im selben Molekül sowohl eine negative als auch eine positive Ladung tragen. Beispiele für bevorzugte zwitterionische Tenside sind die Betaine, die N-Alkyl-N,N-dimethylammonium-glycinate, die N-Acylaminopropyl-N,N-dimethylammoniumglycinate und die 2-Alkyl-3-carboxymethyl-3-hydroxyethylimidazoline mit jeweils 8 bis 24 Kohlenstoffatomen in der Alkylgruppe. Beispiele für bevorzugte ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropyl-glycine, N-Alkyltaurine, N-Alkylsarcosine, 2-Alkylaminopropionsäuren und Alkylaminoessigsäuren mit jeweils 8 bis 24 Kohlenstoffatomen in der Alkylgruppe

Die erfindungsgemäßen Zusammensetzungen, welche als Emulsion, insbesondere als Öl-in-Wasser-Emulsion, formuliert sind, enthalten bevorzugt mindestens einen nichtionischen Öl-in-Wasser-Emulgator mit einem HLB-Wert von mehr als 7 bis 20. Hierbei handelt es sich um dem Fachmann allgemein bekannte Emulgatoren, wie sie beispielsweise in Kirk-Othmer, "Encyclopedia of Chemical Technology", 3. Aufl., 1979, Band 8, Seite 913-916, aufgelistet sind. Für ethoxylierte Produkte wird der HLB-Wert nach der Formel HLB = (100 - L):5 berechnet, wobei L der Gewichtsanteil der lipophilen Gruppen, das heißt der Fettalkyl- oder Fettacylgruppen, in den Ethylenoxidaddukten, ausgedrückt in Gewichtsprozent, ist. In diesem Zusammenhang kann es erfindungsgemäß bevorzugt sein, wenn weiterhin ein Wasser-in-ÖI-Emulgator mit einem HLB-Wert von grösser 1,0 und kleiner/gleich 7,0 eingesetzt wird. Im Rahmen der vorliegenden Erfindung geeignete nichtionische Öl-in-Wasser-Emulgatoren und geeignete nichtionische Wasser-in-ÖI-Emulgatoren sind beispielsweise in der deutschen Offenlegungsschrift DE 10 2006 004 957 A1 beschrieben.

Zur Verdickung der erfindungsgemäßen schweißhemmenden kosmetischen Mittel werden bevorzugt Hydrogel bildende Substanzen eingesetzt, welche ausgewählt sind aus Celluloseethern, vor allem Hydroxyalkylcellulosen, insbesondere Hydroxypropylcellulose, Hydroxypropylmethylcellulose, Hydroxyethylcellulose, Carboxymethylcellulose, Cetylhydroxyethylcellulose, Hydroxybutylmethylcellulose, Methylhydroxyethylcellulose, weiterhin Xanthan-Gum, Sclerotium Gum, Succinoglucanen, Polygalactomannanen, insbesondere Guar-Gums und Johannisbrotkernmehl (Locust Bean Gum), insbesondere Guar-Gum und Locust Bean Gum selbst und den nichtionischen Hydroxyalkylguarderivaten und Johannisbrotkernmehl-Derivaten, wie Hydroxypropylguar, Carboxymethylhydroxypropylguar, Hydroxypropylmethylguar, Hydroxyethylguar und Carboxymethylguar, weiterhin Pectinen, Agar, Carragheen (Carrageenan), Traganth, Gummi arabicum, Karayagummi, Taragummi, Gellan, Gelatine, Casein, Propylenglycolalginat, Alginsäuren und deren Salze, insbesondere Natriumalginat, Kaliumalginat und Calciumalginat, weiterhin Polyvinylpyrrolidonen, Polyvinylalkoholen, Polyacrylamiden, weiterhin - wenn auch weniger bevorzugt - physikalisch (z. B. durch Vorverkleisterung) und/oder chemisch modifizierten Stärken, insbesondere hydroxypropylierten Stärkephosphaten und Octenylstärkesuccinaten und deren Aluminium-, Calcium- oder Natriumsalzen, weiterhin - ebenfalls weniger bevorzugt - Acrylsäure-Acrylat-Copolymeren, Acrylsäure-Acrylamid-Copolymeren, Acrylsäure-Vinylpyrrolidon-Copolymeren, Acrylsäure-Vinylformamid-Copolymeren und Polyacrylaten. Besonders bevorzugte Hydrogelbildner sind ausgewählt aus Celluloseethern, vor allem aus Hydroxyalkylcellulosen, insbesondere aus Hydroxypropylcellulose, Hydroxypropylmethylcellulose, Hydroxyethylcellulose, Carboxymethylcellulose, Cetylhydroxyethylcellulose, Hydroxybutylmethylcellulose und Methylhydroxyethylcellulose, sowie Mischungen hiervon. Bevorzugt wird Hydroxyethylcellulose als Hydrogelbildner eingesetzt.

Um die Verstopfung der Ausführungsgänge der Schweißdrüsen weiter zu unterstützen, kann es von Vorteil sein, den erfindungsgemäßen schweißhemmenden kosmetischen Mitteln mindestens einen Chelatbildner, der ausgewählt ist aus Ethylendiamintetraessigsäure (EDTA) und ihren Salze sowie aus Nitrilotriessigsäure (NTA) und Mischungen dieser Substanzen, in einer Gesamtmenge von 0,01 bis 0,5 Gew.-%, vorzugsweise von 0,02 bis 0,3 Gew.-%, insbesondere von 0,05 bis 0,1 Gew.-%, bezogen auf das Gesamtgewicht des erfindungsgemäßen schweißhemmenden Mittels, zuzusetzen.

Obwohl ein Vorteil der vorliegenden Erfindung darin liegt, dass neben Aluminiumsesquichlorhydrat und Triethylcitrat keine weiteren Antitranspirantwirkstoffe und/oder Deodorantwirkstoffe ebnthalten sein brauchen, kann die Antitranspirantwirkung der erfindungsgemäßen schweißhemmenden kosmetischen Mittel in Ausführungsformen der Erfindung gesteigert werden, wenn mindestens einen Deodorant-Wirkstoff in einer Gesamtmenge von 0,0001 bis 40 Gew.-%, vorzugsweise von 0,2 bis 20 Gew.-%, bevorzugt von 1 bis 15 Gew.-%, insbesondere von 1,5 bis 5 Gew.-%, bezogen auf das Gesamtgewicht des erfindungsgemäßen schweißhemmenden kosmetischen Mittels, enthalten ist. Sofern Ethanol in den erfindungsgemäßen Mitteln eingesetzt wird, gilt dieses im Rahmen der vorliegenden Erfindung nicht als Deodorant-Wirkstoff, sondern als Bestandteil des Trägers. Erfindungsgemäß bevorzugte Deodorant-Wirkstoffe sind ausgewählt aus der Gruppe der (i) Silbersalze; (ii) aromatischen Alkohole, insbesondere 2-Benzylheptan-1-ol sowie Mischungen von 2-Benzylheptan-1-ol und Phenoxyethanol; (iii) 1,2-Alkandiole mit 5 bis 12 Kohlenstoffatomen, jecoch bevorzugt nich Ethylhexylglycerin; (iv) Wirkstoffe gegen Exoesterasen, insbesondere gegen Arylsulfatase, Lipase, beta-Glucuronidase und Cystathion-[beta]-lyase; (v) kationische Phospholipide; (vi) Geruchsabsorber, insbesondere Silicate, wie Montmorillonit, Kaolinit, Ilit, Beidellit, Nontronit, Saponit, Hectorit, Bentonit, Smectit und Talkum, Zeolithe, Zinkricinoleat, Cyclodextrine; (vii) desodorierend wirkenden Ionenaustauscher; (viii) keimhemmenden Mittel; (ix) präbiotisch wirksamen Komponenten; sowie (x) Mischungen dieser Deodorant-Wirkstoffe.

Bevorzugte erfindungsgemäße schweißhemmenden kosmetische Mittel enthalten weiterhin mindestens ein wasserlösliches mehrwertiges C2-9-Alkanol mit 2 bis 6 Hydroxylgruppen und/oder mindestens ein wasserlösliches Polyethylenglycol mit 3 bis 50 Ethylenoxid-Einheiten sowie Mischungen hiervon. Hierunter fallen nicht die vorstehend erwähnten Deodorant-Wirkstoffe in Form von 1,2-Alkandiolen. Bevorzugte Alkanole sind ausgewählt aus 1,2-Propylenglycol, 2-Methyl-1,3-propandiol, Glycerin, 1,2-Butylenglycol, 1,3-Butylenglycol, 1,4-Butylenglycol, Pentylenglycolen wie 1,2-Pentandiol und 1,5-Pentandiol, Hexandiolen wie 1,2-Hexandiol und 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit, cis-1,4-Dimethylolcyclohexan, trans-1,4-Dimethylolcyclohexan, beliebige Isomeren-Gemische von cis- und trans-1,4-Dimethylolcyclohexan, sowie Mischungen der vorgenannten Substanzen. Geeignete wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind.

Gemäß einer weiteren Ausführungsform der vorliegenden Erfindung enthalten die schweißhemmenden kosmetischen Mittel weiterhin mindestens einen hautkühlenden Wirkstoff. Erfindungsgemäß geeignete hautkühlende Wirkstoffe sind beispielsweise Menthol, Isopulegol sowie Mentholderivate, z. B. Menthyllactat, Menthylglycolat, Menthyl Ethyl Oxamate, Menthylpyrrolidoncarbonsäure, Menthylmethylether, Menthoxypropandiol, Menthonglycerinacetal (9-Methyl-6-(1-methylethyl)-1,4-dioxaspiro (4.5)decan-2-methanol), Monomenthylsuccinat, 2-Hydroxymethyl-3,5,5-trimethylcyclohexanol und 5-Methyl-2-(1-methylethyl)cyclohexyl-N-ethyloxamat. Als hautkühlende Wirkstoffe bevorzugt sind Menthol, Isopulegol, Menthyllactat, Menthoxypropandiol, Menthylpyrrolidoncarbonsäure und 5-Methyl-2-(1-methylethyl)cyclohexyl-N-ethyloxamat sowie Mischungen dieser Substanzen, insbesondere Mischungen von Menthol und Menthyllactat, Menthol, Mentholglycolat und Menthyllactat, Menthol und Menthoxypropandiol oder Menthol und Isopulegol.

Weiterhin kann es vorgesehen sein, dass die erfindungsgemäßen schweißhemmenden kosmetischen Mittel ein Treibmittel enthalten. In diesem Fall sind sie als treibgasgetriebenes Aerosol konfektioniert. Bevorzugte Treibmittel (Treibgase) sind Propan, Propen, n-Butan, iso-Butan, isoButen, n-Pentan, Penten, iso-Pentan, iso-Penten, Methan, Ethan, Dimethylether, Stickstoff, Luft, Sauerstoff, Lachgas, 1,1,1,3-Tetrafluorethan, Heptafluoro-n-propan, Perfluorethan, Monochlordifluormethan, 1,1-Difluorethan, Tetrafluoropropene und zwar sowohl einzeln als auch in deren Mischungen. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie Mischungen dieser Treibgase. Es hat sich gezeigt, dass der Einsatz von n-Butan als einzigem Treibgas erfindungsgemäß besonders bevorzugt sein kann. Die Gesamtmenge der Treibmittel beträgt 20 bis 95 Gew.%, vorzugsweise 30 bis 85 Gew.%, insbesondere 40 bis 75 Gew.-%, jeweils bezogen auf das Gesamtgewicht des Antitranspirants, bestehend aus dem erfindungsgemäßen schweißhemmenden kosmetischen Mittel und dem Treibmittel.

Als Hilfsstoffe können erfindungsgemäß weiterhin lipophile Verdickungsmittel eingesetzt werden. Bevorzugt ist das mindestens eine schweißhemmende Aluminiumsalz ungelöst in mindestens einem bei 20 °C und 1.013 hPa flüssigen kosmetischen Öl suspendiert. Zur besseren Anwendbarkeit kann dieser Suspension noch mindestens ein lipophiles Verdickungsmittel als Suspendierhilfe zugesetzt werden. Erfindungsgemäß bevorzugte lipophile Verdickungsmittel sind ausgewählt aus hydrophobierten Tonmineralien und pyrogenen Kieselsäuren.

In einer bevorzugten Ausführungsform enthalten die erfindungsgemäßen Mittel als deodorierenden Wirkstoff mindestens einen aromatischen Alkohol der Struktur (AA-1), 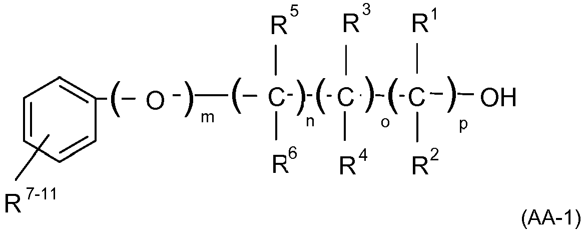 wobei
die Reste R¹ bis R⁶ unabhängig voneinander ein Wasserstoffatom, eine Alkylgruppe mit 1 bis 10 Kohlenstoffatomen, die linear oder verzweigt und substituiert sein können mit OH-Gruppen oder Alkoxy-Gruppen mit 1 bis 5 Kohlenstoffatomen, oder eine Alkenylgruppe mit 2 bis 10 Kohlenstoffatomen, die linear oder verzweigt und substituiert sein können mit OH-Gruppen oder Alkoxy-Gruppen mit 1 bis 5 Kohlenstoffatomen,
die Reste R⁷ bis R¹¹ unabhängig voneinander ein Wasserstoffatom, ein Halogenatom, insbesondere ein Chloratom, oder eine Alkylgruppe mit 1 bis 10 Kohlenstoffatomen, die linear oder verzweigt und substituiert sein können mit OH-Gruppen oder Alkoxy-Gruppen mit 1 bis 5 Kohlenstoffatomen, insbesondere mit einer Methoyxgruppe,
m = 0 oder 1 ist, n, o, p = unabhängig voneinander ganze Zahlen von 0 bis 10 sind, wobei mindestens einer der Werte n, o, p ≠ 0 ist.

Besonders bevorzugte erfindungsgemäße Produkte enthalten mindestens einen Alkohol AA-1, wie vorstehend beschrieben, der ausgewählt ist aus Phenoxyethanol, Anisalkohol, 2-Methyl-5-phenyl-pentan-1-ol, 1,1-Dimethyl-3-phenyl-propan-1-ol, Benzylalkohol, 2-Phenylethan-1-ol, 3-Phenylpropan-1-ol, 4-Phenylbutan-1-ol, 5-Phenylpentan-1-ol, 2-Benzylheptan-1-ol, 2,2-Dimethyl-3-phenylpropan-1-ol, 2,2-Dimethyl-3-(3'-methylphenyl)-propan-1-ol, 2-Ethyl-3-phenylpropan-1-ol, 2-Ethyl-3-(3'-methylphenyl)-propan-1-ol, 3-(3'-Chlorphenyl)-2-ethylpropan-1-ol, 3-(2'-Chlorphenyl)-2-ethylpropan-1-ol, 3-(4'-Chlorphenyl)-2-ethylpropan-1-ol, 3-(3',4'-Dichlorphenyl)-2-ethylpropan-1-ol, 2-Ethyl-3-(2'-methylphenyl)-propan-1-ol, 2-Ethyl-3-(4'-methylphenyl)-propan-1-ol, 3-(3',4'-Dimethyl-phenyl)-2-ethylpropan-1-ol, 2-Ethyl-3-(4'-methoxyphenyl)-propan-1-ol, 3-(3',4'-Dimethoxyphenyl)-2-ethylpropan-1-ol, 2-Allyl-3-phenylpropan-1-ol und 2-n-Pentyl-3-phenylpropan-1-ol sowie Mischungen hiervon. Besonders bevorzugt sind Phenoxyethanol, sowie Mischungen von 2-Benzyl-heptan-1-ol und Phenoxyethanol. Weitere besonders bevorzugte erfindungsgemäße Zusammensetzungen enthalten mindestens einen Alkohol AA-1, wie vorstehend beschrieben, in einer Gesamtmenge von 0,05 - 10 Gew.-%, bevorzugt 0,1 - 5 Gew.-%, besonders bevorzugt 0,2 - 2 Gew.-%, außerordentlich bevorzugt 0,3 - 1,5 Gew.-%, jeweils bezogen auf das Gewicht der Zusammensetzung, ohne ggf. vorhandenes Treibmittel zu berücksichtigen.

Besonders bevorzugte erfindungsgemäße Antitranspirant-Mittel enthalten weiterhin mindestens einen Riechstoff. Die Definition eines Riechstoffs im Sinne der vorliegenden Anmeldung stimmt überein mit der fachmännisch üblichen Definition, wie sie dem RÖMPP Chemie Lexikon, Stand Dezember 2007, entnommen werden kann. Danach ist ein Riechstoff eine chemische Verbindung mit Geruch und/oder Geschmack, der die Rezeptoren der Haarzellen des olfaktorischen Systems erregt (adäquater Reiz). Die hierzu notwendigen physikalischen und chemischen Eigenschaften sind eine niedrige Molmasse von maximal 300 g/mol, ein hoher Dampfdruck, minimale Wasser- und hohe Lipidlöslichkeit sowie schwache Polarität und das Vorliegen mindestens einer osmophoren Gruppe im Molekül. Um flüchtige, niedermolekulare Substanzen, die üblicherweise und auch im Sinne der vorliegenden Anmeldung nicht als Riechstoff, sondern vornehmlich als Lösemittel angesehen und verwendet werden, wie beispielsweise Ethanol, Propanol, Isopropanol und Aceton, von erfindungsgemäßen Riechstoffen abzugrenzen, weisen erfindungsgemäße Riechstoffe eine Molmasse von 74 bis 300 g/mol auf, enthalten mindestens eine osmophore Gruppe im Molekül und weisen einen Geruch und/oder Geschmack auf, das heißt, sie erregen die Rezeptoren der Haarzellen des olfaktorischen Systems.

Als Riechstoffe können Parfüme, Parfümöle oder Parfümölbestandteile eingesetzt werden. Parfümöle bzw. Duftstoffe können erfindungsgemäß einzelne Riechstoffverbindungen, z. B. die synthetischen Produkte vom Typ der Ester, Ether, Aldehyde, Ketone, Alkohole und Kohlenwasserstoffe sein. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Zu den Ethern zählen beispielsweise Benzylethylether und Ambroxan , zu den Aldehyden z.B. die linearen Alkanale mit 8 - 18 C-Atomen, Citral, Citronellal, Citronellyloxy-acetaldehyd, Cyclamenaldehyd, Lilial und Bourgeonal, zu den Ketonen z.B. die Jonone, alpha-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen.

Besonders bevorzugte erfindungsgemäße Antitranspirant-Mittel enthalten mindestens einen Riechstoff in einer Gesamtmenge von 0,00001 bis 10 Gew.-%, bevorzugt 0,5 - 7 Gew.-%, außerordentlich bevorzugt 1 - 6 Gew.-%, jeweils bezogen auf das Gesamtgewicht des Mittels, ohne ggf. vorhandenes Treibmittel zu berücksichtigen.

Die erfindungsgemäßen Mittel enthalten die Inhalts- bzw. Wirkstoffe in einem kosmetisch akzeptablen Träger.

Bevorzugte kosmetisch akzeptable Träger sind wässrige, alkoholische oder wässrig-alkoholische

Medien mit vorzugsweise mindestens 10 Gew.-% Wasser, berechnet auf das Gesamtgewicht des Mittels.

Besonders bevorzugt enthält der erfindungsgemäße kosmetische Träger Wasser, insbesondere in der Menge, dass das kosmetische Mittel, berechnet auf das Gesamtgewicht des Mittels, mindestens 10 Gew.-%, insbesondere mindestens 20,0 Gew.-%, am bevorzugtesten mindestens 30 Gew.-% Wasser enthält.

Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Beispiele für wasserlösliche Lösungsmittel als Cosolvens sind Glycerin und/oder Ethylenglykol und/oder 1,2-Propylenglykol in einer Menge von 0 bis 30 Gew.-% bezogen auf das gesamte Mittel.

Die Applikation des erfindungsgemäßen schweißhemmenden kosmetischen Mittels kann mittels verschiedener Verfahren erfolgen. Gemäß einer bevorzugten Ausführungsform ist das schweißhemmende kosmetische Mittel als Spray-Applikation konfektioniert. Die Spray-Applikation erfolgt mit einer Sprühvorrichtung, welche in einem Behälter eine Füllung aus dem erfindungsgemäßen flüssigen, viskos-fließfähigen, suspensionsförmigen oder pulverförmigen schweißhemmenden kosmetischen Mittel enthält. Die Füllung kann unter dem Druck eines Treibmittels stehen (Druckgasdosen, Druckgaspackungen, Aerosolpackungen), oder es kann sich um einen mechanisch zu bedienenden Pumpzerstäuber ohne Treibgas (Pumpsprays/ Quetschflasche) handeln. Die Behälter weisen eine Entnahmevorrichtung auf, bevorzugt in Gestalt von Ventilen, welche die Entnahme des Inhalts als Nebel, Rauch, Schaum, Pulver, Paste oder Flüssigkeitsstrahl ermöglichen. Als Behälter für die Sprühvorrichtungen kommen vor allem zylindrische Gefäße aus Metall (Aluminium, Weißblech, Rauminhalt bevorzugt maximal 1.000 ml), geschütztem bzw. nichtsplitterndem Glas oder Kunststoff (Rauminhalt bevorzugt maximal 220 ml) bzw. splitterndem Glas oder Kunststoff (Rauminhalt bevorzugt 50 bis 400 ml) in Frage. Cremeförmige, gelförmige, pastöse und flüssige Mittel können z.B. in Pump-, Spray- oder Quetschspendern abgepackt sein, insbesondere auch in Mehrkammer-Pump-, Mehrkammer-Spray- oder Mehrkammer-Quetschspendern. Die Verpackung für die erfindungsgemäßen Mittel kann undurchsichtig, aber auch transparent oder transluzent sein.

Das schweißhemmende kosmetische Mittel ist bevorzugt als Stift, Soft Solid, Creme, Roll-on, Dibenzylidenalditol-basiertes Gel, loses oder kompaktes Puder konfektioniert. Die Formulierung der erfindungsgemäßen schweißhemmenden kosmetischen Mittel in einer bestimmten Darreichungsform, wie beispielsweise einem Antitranspirant-Roll-on, einem Antitranspirantstift oder einem Antitranspirantgel, richtet sich bevorzugt nach den Anforderungen des Verwendungszwecks. Je nach Verwendungszweck können die erfindungsgemäßen schweißhemmenden kosmetischen Mittel daher in fester, halbfester, flüssiger, disperser, emulgierter, suspendierter, gelförmiger, mehrphasiger oder puderförmiger Form vorliegen. Unter den Begriff der Flüssigkeit fallen im Sinne der vorliegenden Erfindung auch jegliche Arten von Festkörperdispersionen in Flüssigkeiten. Weiterhin werden unter mehrphasigen erfindungsgemäßen schweißhemmenden kosmetischen Mitteln im Sinne der vorliegenden Erfindung Mittel verstanden, welche mindestens 2 verschiedene Phasen mit einer Phasentrennung aufweisen und bei welchen die Phasen horizontal, also übereinander, oder vertikal, also nebeneinander, angeordnet sein können.

Die Applikation kann beispielsweise mit einem Rollkugelapplikator erfolgen. Solche Roller weisen eine in einem Kugelbett gelagerte Kugel auf, welche durch Bewegung über eine Oberfläche bewegt werden kann. Dabei nimmt die Kugel etwas von dem zu verteilenden erfindungsgemäßen schweißhemmenden kosmetischen Mittel auf und befördert dieses an die zu behandelnde Oberfläche. Die Verpackung für die erfindungsgemäßen Mittel kann, wie zuvor ausgeführt, undurchsichtig, transparent oder transluzent sein.

Weiterhin ist es auch möglich, die erfindungsgemäßen schweißhemmenden kosmetischen Mittel mittels eines festen Stifts in Form einer festen Emulsion zu applizieren.

Es kann erfindungsgemäß jedoch auch bevorzugt sein, dass das schweißhemmende kosmetische Mittel auf und/oder in einem wegwerfbaren Substrat, ausgewählt aus der Gruppe von Tüchern, Pads und Bauschen, enthalten ist. Besonders bevorzugt sind Feuchttücher, d.h. für den Anwender vorgefertigte, bevorzugt einzeln abgepackte, Feuchttücher, wie sie z. B. aus dem Bereich der Glasreinigung oder aus dem Bereich der feuchten Toilettenpapiere wohlbekannt sind. Solche Feuchttücher, die vorteilhafter Weise auch Konservierungsstoffe enthalten können, sind mit einem erfindungsgemäßen schweißhemmenden kosmetischen Mittel imprägniert oder beaufschlagt und bevorzugt einzeln verpackt. Sie können z. B. als Deodorant-Tuch eingesetzt werden, was besonders interessant für den Gebrauch unterwegs ist. Bevorzugte Substratmaterialien sind ausgewählt aus porösen flächigen Tüchern. Sie können aus einem faserigen oder zellulären flexiblen Material bestehen, das ausreichend mechanische Stabilität und gleichzeitig Weichheit zur Anwendung auf der Haut aufweist. Zu diesen Tüchern gehören Tücher aus gewebten und ungewebten (Vlies) synthetischen und natürlichen Fasern, Filz, Papier oder Schaumstoff, wie hydrophilem Polyurethanschaum. Erfindungsgemäß bevorzugte deodorierende oder schweißhemmende Substrate können durch Tränken oder Imprägnierung oder auch durch Aufschmelzen eines erfindungsgemäßen schweißhemmenden kosmetischen Mittels auf ein Substrat erhalten werden.

### Tabellarische Übersicht

Die Zusammensetzung einiger bevorzugter kosmetischer Mittel kann den folgenden Tabellen entnommen werden (Angaben in Gew.-% bezogen auf das Gesamtgewicht des kosmetischen Mittels sofern nicht anders angegeben, ohne ggf. verwendetes Treibmittel zu berücksichtigen).

| | Formel 1 | Formel 2 | Formel 3 | Formel 4 |
|---|---|---|---|---|
| Triethylcitrat | 0,1 bis 7,0 | 0,5 bis 5,0 | 0,5 bis 3,0 | 1,0 bis 2,0 |
| Aluminiumsesquichlorhydrat | 5,0 bis 15,0 | 6,0 bis 14,0 | 7,0 bis 13,0 | 8,0 bis 12,0 |
| Misc | add 100 | add 100 | add 100 | add 100 |

| | Formel 1a | Formel 2a | Formel 3a | Formel 4a |
|---|---|---|---|---|
| Triethylcitrat | 0,1 bis 7,0 | 0,5 bis 5,0 | 0,5 bis 3,0 | 1,0 bis 2,0 |
| Aluminiumsesquichlorhydrat | 5,0 bis 15,0 | 6,0 bis 14,0 | 7,0 bis 13,0 | 8,0 bis 12,0 |
| Chelatbildner, z. B. EDTA | 0,01 bis 0,5 | 0,02 bis 0,4 | 0,03 bis 0,3 | 0,05 bis 0,1 |
| Misc | add 100 | add 100 | add 100 | add 100 |

| | Formel 1b | Formel 2b | Formel 3b | Formel 4b |
|---|---|---|---|---|
| Triethylcitrat | 0,1 bis 7,0 | 0,5 bis 5,0 | 0,5 bis 3,0 | 1,0 bis 2,0 |
| Aluminiumsesquichlorhydrat | 5,0 bis 15,0 | 6,0 bis 14,0 | 7,0 bis 13,0 | 8,0 bis 12,0 |
| Phenoxyethanol | 0,05 bis 10,0 | 0,1 bis 3,0 | 0,3 bis 2,0 | 0,5 bis 1,5 |
| Misc | add 100 | add 100 | add 100 | add 100 |

| | Formel 1c | Formel 2c | Formel 3c | Formel 4c |
|---|---|---|---|---|
| Triethylcitrat | 0,1 bis 7,0 | 0,5 bis 5,0 | 0,5 bis 3,0 | 1,0 bis 2,0 |
| Aluminiumsesquichlorhydrat | 5,0 bis 15,0 | 6,0 bis 14,0 | 7,0 bis 13,0 | 8,0 bis 12,0 |
| Chelatbildner, z. B. EDTA | 0,01 bis 0,5 | 0,02 bis 0,4 | 0,03 bis 0,3 | 0,05 bis 0,1 |
| Phenoxyethanol | 0,05 bis 10,0 | 0,1 bis 3,0 | 0,3 bis 2,0 | 0,5 bis 1,5 |
| Misc | add 100 | add 100 | add 100 | add 100 |

Unter "Misc" ist erfindungsgemäß ein kosmetischer Träger zu verstehen, und ggf. weitere übliche Bestandteile von antitranspiranten Mitteln. Es sind dabei ausdrücklich auch Ausführungsformen umfasst, bei denen "Misc" kein Ethylhexylglycerin enthält, sowie Ausführungsformen, bei denen "Misc" kein Dipropylenglykol enthält, und Ausführungsformen, bei denen "Misc" keine weiteren antitranspiranten Wirkstoffe enthält, sowie Ausführungsformen, bei denen "Misc" eines oder mehrere von Ethylhexylglycerin, Dipropylenglykol und weiteren antitranspiranten Wirkstoffe nicht enthält.

### Ausführungsbeispiele

Die nachfolgenden Formulierungsbeispiele sollen den Gegenstand der Erfindung erläutern, ohne ihn hierauf zu beschränken.

Die nachfolgend in der Tabelle beschriebenen kosmetischen Zusammensetzungen wurden als Emulsionen hergestellt.

Antitranspirant Roll-ons (Angaben in Gew.-%)

| Komponente bzw. INCI | Hersteller | 1 | 2 | 3 | 4 |
|---|---|---|---|---|---|
| PPG-15 Stearyl Ether | | 0,5 | 0,5 | 0,5 | 0,5 |
| Steareth-2 | | 2,4 | 2,4 | 2,4 | 2,4 |
| Steareth-21 | | 1,5 | 1,5 | 1,5 | 1,5 |
| Triethyl Citrate (citroflex 2) | Vertellus Specialities Inc. | 1,0 | 1,0 | 1,5 | 2,0 |
| Aluminum, Sesquichlorohydrate (Reach 301L, 40 % in Wasser) | | 20,0 | 25,0 | 30,0 | 30,0 |
| EDTA BX Powder | BASF | 0,1 | 0,1 | 0,1 | 0,1 |
| Parfum | | 1,0 | 1,0 | 1,0 | 1,0 |
| Wasser | | 73,5 | 68,5 | 64,0 | 63,5 |

Antitranspirant-Aerosole (Angaben in Gew.-%)

| Komponenten bzw. INCI | Hersteller | 5 | 6 | 7 |
|---|---|---|---|---|
| Cyclopentasiloxane (Xiameter PMX-0245) | Dow Corning | 10,7 | 10,7 | 10,7 |
| 25% PEG/PPG-18/18 Dimethicone / 85% Dimethicone 5 cts (Dow Corning ES-5227 DM) | Dow Corning | 5,6 | 5,6 | 5,6 |
| Isopropyl Myristate | BASF | 6,6 | 6,6 | 6,6 |
| Propylenglycol | | 23,4 | 23,4 | 23,4 |
| Phenoxyethanol | | 0,5 | 0,5 | 0,5 |
| Aluminum, Sesquichlorohydrate (Reach 301L, 40 % in Wasser) | Summit Reheis | 20,0 | 25,0 | 30,0 |
| Triethyl Citrate (citroflex 2) | Vertellus Specialities Inc. | 1,0 | 1,0 | 2,0 |
| Parfum | | 2,5 | 2,5 | 2,5 |
| Wasser | | 29,7 | 24,7 | 18,7 |

Die Rezepturen 5, 6 und 7 wurden im Gewichtsverhältnis von 1:4 mit dem Treibmittel Propan/Butan (15/85) in Aerosoldosen abgefüllt.

Der bei Normalbedingungen mit dem pH Meter pH730 von inoLab gemessene pH der erfindungsgemäßen kosmetischen Zusammensetzungen 1 bis 7 lag jeweils zwischen 3,5 und 4,0.

### Ergebnisse:

Bei Anwendung der Rezepturen wurde von Testpersonen eine sehr gute Hautverträglichkeit, wenige Rückstände und Flecken auf der Haut und Textilien und ein verbessertes, weniger klebriges Hautgefühl beobachtet.

Weiterhin wurde bei Anwendung des erfindungsgemäßen kosmetischen Mittels (Formulierung E unten) auf der Hautoberfläche eine Absenkung des pH-Werts der Haut beobachtet. Die Vergleichsformulierung (V) mit 26% Aluminium Chlorohydrat ohne Citronensäuretriethylester zeigt in diesem Tesdesign keine messbare pH-Absenkung. Die Formulierungen wurden unter kontrollierten Bedingungen an 20 Testpersonen in der Achsel aufgetragen. Zuvor wurden für 5 Tage keine Deoprodukte angewendet und die Achseln nur mit neutraler Seife gewaschen. Die Messungen erfolgten mit dem SKIN pH-Meter 905 (Courage + Khazaka electronic GmbH). Die beiden Produkte wurden an unterschiedlichen Probandenkollektiven getestet. Daher konnte nur der pH-Shift, nicht aber die absoluten Werte zum jeweiligen Zeitpunkt verglichen werden.

| Einfluss unterschiedlicher Testformulierungen auf den pH-Wert der Haut in der Achsel**Komponente bzw. INCI** | **Hersteller** | **E** | **V** |
|---|---|---|---|
| PPG-15 Stearyl Ether | | 0,5 | 0,5 |
| Steareth-2 | | 2,4 | 2,4 |
| Steareth-21 | | 1,5 | 1,5 |
| Triethyl Citrate (citroflex 2) | Vertellus Specialities Inc. | 1,0 | - |
| Aluminum, Sesquichlorohydrate (Reach 301L, 40 % in Wasser) | Summit Reheis | 25,0 | - |
| Aluminum Chlorohydrate (Locron L, 50 % in Wasser) | Clariant | - | 26,0 |
| EDTA BX Powder | BASF | 0,1 | 0,1 |
| Wasser | | 69,5 | 69,5 |

| **Axillary Skin pH** | | | |
|---|---|---|---|
| | T 0 min | 6,0 | 5,3 |
| | T 20 min | 5,8 | 5,3 |
| | T 40 min | 5,8 | 5,4 |
| | T 60 min | 5,7 | 5,4 |

## Patentansprüche

1. Antitranspirantes kosmetisches Mittel in Form einer wässrigen Emulsion, welches enthält:
- Aluminiumsesquichlorhydrat und
- Citronensäuretriethylester,
• wobei das kosmetische Mittel einen pH-Wert von 3,5 bis 4,0 aufweist und
• wobei das Aluminiumsesquichlorhydrat in einer Menge von 5,0 bis 15,0 Gew.-% enthalten ist und der Citronensäuretriethylester in einer Menge von 0,1 bis 7 Gew.-% enthalten ist, wobei sich die Gew.-%-Angaben jeweils auf das Gesamtgewicht des erfindungsgemäßen Mittels beziehen, ohne ggf. vorhandenes Treibmittel zu berücksichtigen und
• wobei das Aluminiumsesquichlorhydrat ein molares Al/Cl-Verhältnis von 1,5:1 bis 1,8:1 aufweist.

2. Kosmetisches Mittel nach Anspruch 1, wobei das Aluminiumsesquichlorhydrat in einer Menge von 8,0 bis 12,0 Gew.-% enthalten ist und der Citronensäuretriethylester in einer Menge von 0,5 bis 2,0 Gew.-% enthalten ist, wobei sich die Gew.-%-Angaben jeweils auf das Gesamtgewicht des erfindungsgemäßen Mittels beziehen, ohne ggf. vorhandenes Treibmittel zu berücksichtigen.

3. Kosmetisches Mittel nach einem der vorhergehenden Ansprüche, wobei das kosmetische Mittel kein Ethylhexylglycerin enthält.

4. Kosmetisches Mittel nach einem der vorhergehenden Ansprüche, wobei das kosmetische Mittel kein Dipropylenglykol enthält.

5. Kosmetisches Mittel nach einem der vorhergehenden Ansprüche, wobei das kosmetische Mittel weiterhin Phenoxyethanol enthält.

6. Kosmetisches Mittel nach einem der vorhergehenden Ansprüche, wobei das kosmetische Mittel als Ol-in-Wasser-Emulsion vorliegt.

7. Verfahren zur nicht-therapeutischen, kosmetischen schweißhemmenden Behandlung des Körpers, bei dem ein antitranspirantes kosmetisches Mittel nach einem der vorhergehenden Ansprüche auf die Haut, insbesondere die Haut der Achselhöhlen, aufgebracht wird.

8. Nichttherapeutische, kosmetische Verwendung einer wässrigen Emulsion, nach einem der Ansprüche 1 bis 6 zur Reduzierung und/oder Verhinderung von Schweiß, insbesondere Achselschweiß.

## Claims

1. An antiperspirant cosmetic agent in the form of an aqueous emulsion, containing:
- aluminum sesquichlorohydrate and
- citric acid triethyl ester,
• wherein the cosmetic agent has a pH of 3.5 to 4.0 and
• wherein the aluminum sesquichlorohydrate is contained in an amount of from 5.0 to 15.0 wt.% and the citric acid triethyl ester is contained in an amount of from 0.1 to 7 wt.%, wherein the amounts given in wt.% are based in each case on the total weight of the agent according to the invention, without taking into account the optionally present propellant and
• wherein the aluminum sesquichlorohydrate has a molar AI/CI ratio of 1.5:1 to 1.8:1.

2. The cosmetic agent according to claim 1, wherein the aluminum sesquichlorohydrate is contained in an amount of from 8.0 to 12.0 wt.% and the citric acid triethyl ester is contained in an amount of from 0.5 to 2.0 wt.%, wherein the amounts given in wt.% are based in each case on the total weight of the agent according to the invention, without taking into account the optionally present propellant.

3. The cosmetic agent according to one of the preceding claims, wherein the cosmetic agent does not contain ethylhexylglycerin.

4. The cosmetic agent according to one of the preceding claims, wherein the cosmetic agent does not contain dipropylene glycol.

5. The cosmetic agent according to one of the preceding claims, wherein the cosmetic agent also contains phenoxyethanol.

6. The cosmetic agent according to one of the preceding claims, wherein the cosmetic agent is an oil-in-water emulsion.

7. A method for non-therapeutic, cosmetic antiperspirant treatment of the body, wherein an antiperspirant cosmetic agent according to one of the preceding claims is applied to the skin, in particular the underarm skin.

8. The non-therapeutic, cosmetic use of an aqueous emulsion according to one of claims 1 to 6 for reducing and/or preventing sweat, in particular underarm sweat.

## Revendications

1. Agent cosmétique anti-transpirant sous forme d'émulsion aqueuse contenant :
- du sesquichlorohydrate d'aluminium et
- du citrate de triéthyle,
• dans lequel l'agent cosmétique a un pH valant de 3,5 à 4,0 et
• dans lequel le sesquichlorohydrate d'aluminium est présent en une quantité allant de 5,0 à 15,0 % en poids et le citrate de triéthyle est présent en une quantité allant de 0,1 à 7 % en poids, les données exprimées en % en poids se rapportant respectivement au poids total de l'agent selon l'invention, le cas échéant sans tenir compte de l'agent propulseur existant et
• dans lequel le sesquichlorohydrate d'aluminium a un rapport molaire Al/Cl valant de 1,5:1 à 1,8:1.

2. Agent cosmétique selon la revendication 1, dans lequel le sesquichlorohydrate d'aluminium est présent en une quantité allant de 8,0 à 12,0 % en poids et le citrate de triéthyle est présent en une quantité allant de 0,5 à 2,0 % en poids, les données exprimées en % en poids se rapportant respectivement au poids total de l'agent selon l'invention, le cas échéant sans prendre en compte l'agent propulseur présent.

3. Agent cosmétique selon l'une des revendications précédentes, dans lequel l'agent cosmétique ne contient pas d'éthylhexylglycérol.

4. Agent cosmétique selon l'une des revendications précédentes, dans lequel l'agent cosmétique ne contient pas de dipropylène glycol.

5. Agent cosmétique selon l'une des revendications précédentes, dans lequel l'agent cosmétique contient en outre du phénoxyéthanol.

6. Agent cosmétique selon l'une des revendications précédentes, dans lequel l'agent cosmétique se présente sous la forme d'une émulsion huile-dans-eau.

7. Procédé pour le traitement anti-transpirant cosmétique, non thérapeutique, du corps humain, dans lequel un agent cosmétique anti-transpirant selon l'une des revendications précédentes est appliqué sur la peau, en particulier sur la peau des aisselles.

8. Utilisation cosmétique, non thérapeutique, d'une émulsion aqueuse, selon l'une des revendications 1 à 6 pour la réduction et/ou la prévention de la transpiration, en particulier la transpiration des aisselles.
